# EUROPEAN PATENT SPECIFICATION

(11) **EP 1 171 036 B1**
(45) Date of publication and mention of the grant of the patent: **27.07.2005**
(21) Application number: 01905988.0
(22) Date of filing: 22.02.2001
(51) Int. Cl.: A61B 5/15

(54) **IMPROVEMENTS RELATING TO SKIN PRICKERS**
VERBESSERUNG AN HAUTEINSCHNITTVORRICHTUNGEN
AMELIORATIONS RELATIVES AUX DISPOSITIFS POUR PIQUER LA PEAU

(30) Priority: 22.02.2000 GB 0003991
(43) Date of publication of application: 16.01.2002
(73) Proprietor: OWEN MUMFORD LIMITED, Woodstock, Oxford OX20 1TU (GB)
(72) Inventor: DAVISON, Glenn, Banbury, Oxon OX16 OUG (GB)
(74) Representative: James, Michael John Gwynne
(86) International application number: PCT/GB2001/000780
(87) International publication number: WO 2001/062150

(56) References cited:
- EP-A- 0 555 554
- WO-A-85/04089
- WO-A-97/08986
- US-A- 5 026 388

## Description

This invention relates to skin prickers.

There are many different kinds of skin prickers, mostly being spring loaded devices which fire disposable lancets. Some pricking devices, such as disclosed in US-A-5 026 388 are entirely disposable after a single use. But whatever form they take, there is no avoidance of the fact that they puncture the skin of the user and inflict pain. This may be trivial and transitory, but many users would welcome its reduction, while accepting its elimination is not really possible.

It is the aim of this invention, if not to reduce the pain, at least to divert attention from it.

According to the present invention there is provided a skin pricker comprising a housing with means for projecting a lancet therein forwards, momentarily to expose the tip of the lancet through an aperture in the nose of the housing, wherein the aperture is in a platform generally perpendicular to the direction of motion of the lancet, characterised in that projections are provided on the platform to press into the user's skin adjacent the point to be punctured by the lancet.

The platform will usually be circular, with the aperture at the centre, and the projections will conveniently be in an annular array around the aperture. Coned studs appear to be effective, but other shapes such as serrations or elongate teeth arranged like the spokes of a wheel could work. In any event, their shape, size and spacing should be such that, when pressed against the skin, they should be felt by the user to the point of slight discomfort, but not be painful. The effect should be to confuse the nerves in the region of the impending prick so that, when that prick is made, it is less noticeable than it would otherwise be.

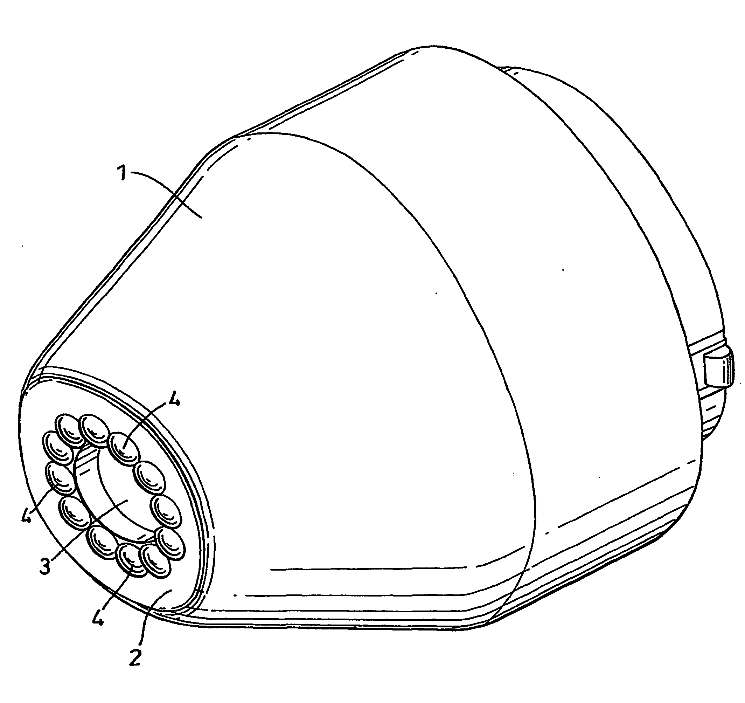

A proposed arrangement is shown, by way of example, in the accompanying drawing, which is a perspective view of the nose cone of a lancet firing device, or skin pricker.

The nose cone 1 terminates at its forward end in an annular platform 2 at right angles to the axis of the cone. The tip of the lancet (not shown) is projected through the central aperture 3. An annular array of studs 4 surrounds the aperture, standing slightly proud of the platform 2. Each stud 4 is of shallow conical form with the sharpness of its tip dulled by a very slight rounding.

Thus, in use, there will be twelve (in the example shown) points pressing into the skin around the prick point.

It will be understood that the number and arrangement of the studs may vary, and that their size and shape may differ. For example, the array could zigzag as it encircles the aperture, with generally pyramidal studs. Alternatively, there might be two arrays, one outside the other, with different numbers and spacing of the studs in the two arrays, and perhaps differently sized and shaped studs in the outer array compared with those in the inner array. There is also the option referred to above of serrations or teeth.

Given that a typical aperture 3 might be 3-4mm in diameter, the drawing indicates the size of studs that have been found to be effective. But that is not intended to be limiting, and it is quite possible that a range of stud sizes may be provided, the user being able to choose, by trial and error, which size and/or pattern is most beneficial.

## Claims

1. A skin pricker comprising a housing with means for projecting a lancet therein forwards, momentarily to expose the tip of the lancet through an aperture (3) in the nose (1) of the housing, wherein the aperture (3) is in a platform (2) generally perpendicular to the direction of motion of the lancet **characterised in that** the platform is in the form of an end plate and the projections (4) are provided on the platform in the form of studs surrounding the aperture in the platform (2) to press into the user's skin adjacent the point to be punctured by the lancet.

2. A skin pricker as claimed in Claim 1, **characterised in that** the platform (2) is circular, with the aperture (3) at the centre, and the projections (4) are in an annular array around the aperture.

3. A skin pricker as claimed in Claim 1 or 2, **characterised in that** the projections are tapered studs (4).

## Patentansprüche

1. Vorrichtung zum Durchstechen der Haut umfassend ein Gehäuse mit einer Einrichtung, um eine Lanzette hierin augenblicklich nach vorne herausragen zu lassen zur Freigabe der Spitze der Lanzette durch eine Öffnung (3) in der Nase (1) des Gehäuses, wobei die Öffnung (3) sich in einer Plattform (2) befindet, die im wesentlichen senkrecht zur Bewegungsrichtung der Lanzette angeordnet ist,
**dadurch gekennzeichnet,**
**dass** die Plattform eine Endplatte bildet und die Vorsprünge (4) auf der Plattform vorgesehen sind in der Form von Zapfen, die die Öffnung in der Plattform (2) umgeben zum Einpressen in die Haut des Benutzers angrenzend an den durch die Lanzette zu durchstechenden Punkt.

2. Vorrichtung zum Durchstechen der Haut gemäß Anspruch 1, **dadurch gekennzeichnet, dass** die Plattform (2) kreisförmig ausgebildet ist mit der Öffnung (3) in der Mitte, während die Vorsprünge (4) sich in einer ringförmigen Anordnung um die Öffnung herum befinden.

3. Vorrichtung zum Durchstechen der Haut gemäß Anspruch 1 oder 2, **dadurch gekennzeichnet, dass** die Vorsprünge als sich zur Spitze verjüngende Zapfen (4) ausgebildet sind.

## Revendications

1. Dispositif à piquer la peau comprenant un boîtier avec un moyen pour projeter vers l'avant une lancette se trouvant dans celui-ci, pour exposer momentanément l'extrémité de la lancette à travers une ouverture (3) dans le nez (1) du boîtier, dans lequel l'ouverture (3) se situe dans une plate-forme (2) généralement perpendiculaire à la direction de mouvement de la lancette, **caractérisé par le fait que** la plate-forme se présente sous la forme d'une plaque d'extrémité et que des projections (4) sont disposées sur la plate-forme sous la forme de boutons entourant l'ouverture dans la plate-forme (2) pour s'enfoncer dans la peau de l'utilisateur au voisinage du point devant être piqué par la lancette.

2. Dispositif à piquer la peau selon la revendication 1, **caractérisé par** le fait la plate-forme (2) est circulaire, avec l'ouverture (3) au centre, et les projections (4) sont dans un arrangement annulaire autour de l'ouverture.

3. Dispositif à piquer la peau selon l'une des revendications 1 ou 2, **caractérisé par le fait que** les projections sont des boutons coniques (4).
